Europäisches Patentamt

European Patent Office

Office européen des brevets

(11) Veröffentlichungsnummer: **0 319 648 B1**

(19)

(12) **EUROPÄISCHE PATENTSCHRIFT**

(45) Veröffentlichungstag der Patentschrift: **15.01.92**

(21) Anmeldenummer: **88111730.3**

(22) Anmeldetag: **21.07.88**

(51) Int. Cl.⁵: **A61M 5/14**

(54) **Verfahren zur Überwachung des Betriebs einer Infusionsspritzenpumpe.**

(30) Priorität: **10.12.87 DE 3741802**

(43) Veröffentlichungstag der Anmeldung:
**14.06.89 Patentblatt 89/24**

(45) Bekanntmachung des Hinweises auf die
Patenterteilung:
**15.01.92 Patentblatt 92/03**

(84) Benannte Vertragsstaaten:
**AT CH DE ES FR GB IT LI NL SE**

(56) Entgegenhaltungen:
**WO-A-87/05224**
**FR-A- 2 518 410**
**GB-A- 2 153 445**

(73) Patentinhaber: **B. Braun Melsungen AG**
**Carl-Braun Strasse**
**W-3508 Melsungen(DE)**

(72) Erfinder: **Steger, Jürgen**
**Lohrer Strasse 11**
**W-3582 Felsberg(DE)**
Erfinder: **Seidel, Franz**
**Kirchweg 15b**
**W-3500 Kassel(DE)**
Erfinder: **Heitmeier, Rolf**
**Goethestrasse 8**
**W-3507 Baunatal(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

## Beschreibung

Die Erfindung betrifft ein Verfahren nach dem Oberbegriff des Patentanspruchs 1.

Bekannt sind Druckinfusionsapparate für medizinische Anwendungen, die zum Applizieren von Medikamenten eine Spritze ausdrücken (DE-C-3 340 511). Derartige Apparate weisen eine Einrichtung auf, die bei Überschreiten einer bestimmten Kolbenkraft der Spritze zur Erzeugung eines Alarmsignals bzw. zur Abschaltung führt. Die Erzeugung des Signals erfolgt bei dem bekannten Druckinfusionsapparat dadurch, daß der feste Halter, an dem der Spritzenzylinder abgestützt ist, eine Verschiebebewegung in Richtung auf einen Schalter ausführen kann und von einer Feder in Richtung auf den angetriebenen Halter vorgespannt ist. Bei Okklusion der vom Spritzenzylinder zum Patienten führenden Flüssigkeitsleitung oder beim Anstoßen des Kolbens gegen die Zylinderbrust wird durch die auf den Spritzenzylinder ausgeübte Kraft der Halter gegen die Wirkung der Feder verschoben, so daß der Schalter betätigt und Alarm ausgelöst wird. Dieses Prinzip hat den Nachteil, daß eine aufwendige mechanische Konstruktion erforderlich ist und daß die Ansprechgenauigkeit gering ist. Das bekannte System ist mit einem erheblichen Bolusvolumen oder Flüssigkeitsrestvolumen (Weg x Kolbenfläche) behaftet. Ferner ist nachteilig, daß die Signalerzeugung in Abhängigkeit von der Kolbenkraft erfolgt. Bei Spritzen mit großem Durchmesser ist die Reibkraft zwischen Kolben und Spritzenzylinder größer als bei Spritzen mit kleinem Durchmesser. Außerdem erfordern Spritzen mit großem Durchmesser eine größere Vorschubkraft des Kolbens, um einen bestimmten für das Austreiben der Flüssigkeit erforderlichen Druck zu erzeugen, als Spritzen mit kleinem Durchmesser. Bei größeren Spritzen wird daher die Kraft, bei der der Schalter anspricht, früher erreicht als bei Spritzen mit kleinem Durchmesser.

Ferner ist es bekannt, in die an den Spritzenzylinder angeschlossene Infusionsleitung eine Druckmeßdose einzubauen, um den Flüssigkeitsdruck zur Abschaltung der Infusionsspritzenpumpe auszuwerten. Die Spritze und die Infusionsleitung stellen jedoch Einmalartikel dar, die nach Gebrauch fortgeworfen werden. Die Kosten dieser Einmalartikel werden durch die Druckmeßeinrichtung wesentlich erhöht.

Die bekannten Verfahren messen den Flüssigkeitsdruck nicht quantitativ, sondern überwachen diesen Druck bzw. die Kolbenkraft lediglich nach Art eines Grenzwertdetektors.

Der Erfindung liegt die Aufgabe zugrunde, ein Verfahren der im Oberbegriff des Patentanspruchs 1 angegebenen Art zu schaffen, das unabhängig von der Form und Größe der Spritze reproduzierbare Ansprechverhältnisse liefert, ohne daß ein Drucksensor in dem als Einmalartikel ausgebildeten Flüssigkeitssystem benötigt würde.

Die Lösung dieser Aufgabe erfolgt erfindungsgemäß mit den im kennzeichnenden Teil des Patentanspruchs 1 angegebenen Merkmalen.

Bei dem erfindungsgemäßen Verfahren wird der im Inneren des Spritzenzylinders herrschende Flüssigkeitsdruck quantitativ gemessen, indem die Kraft die einem der Halter der Infusionsspritzenpumpe von der Spritze entgegengesetzt wird, gemessen wird. Von dieser Kraft wird die Reibkraft subtrahiert und das Subtraktionsergebnis wird mit einem der Größe der Kolbenfläche entsprechenden Wert multipliziert. Die Reibkraft und die Kolbenfläche der Spritze sind Parameter, die dem Rechenwerk, z.B. einem Mikroprozessor, vorgegeben werden. Diese Parameter können entweder einzeln eingegeben werden oder es wird eine Kenngröße für die Spritze in den Mikroprozessor eingegeben, aus der der Mikroprozessor die Werte von Reibkraft und Kolbenfläche ermittelt. Das Rechenwerk kann auch aus einer Digital- oder Analogschaltung bestehen.

Ein wesentlicher Vorteil der Erfindung besteht darin, daß die Erzeugung des Signals zur Abschaltung oder zur Alarmgabe nicht in Abhängigkeit von der Kolbenkraft, sondern in Abhängigkeit vom Flüssigkeitsdruck erfolgt, ohne daß ein Eingriff in das aus Spritze und Infusionsleitung bestehende Flüssigkeitssystem erforderlich wäre. Spritze und Infusionsleitung können daher aus billigen Einmalartikeln bestehen. Da der Flüssigkeitsdruck quantitativ errechnet wird, kann der obere Grenzwert beliebig eingestellt oder verändert werden, indem ein entsprechender neuer Grenzwert in das Rechenwerk eingegeben wird. Es sind daher keine mechanischen Verstellungen an der Infusionsspritzenpumpe erforderlich, um die Bedingungen der Signalerzeugung zu verändern. Die druckabhängige Signalerzeugung hat den Vorteil, daß eine bestimmte Druckdifferenz, d.h. eine Erhöhung des Drucks gegenüber dem Betriebsdruck, zur Alarmerzeugung bzw. Abschaltung der Infusionsspritzenpumpe führt, unabhängig von den Querschnittsabmessungen der Spritze. Eine nach dem erfindungsgemäßen Verfahren arbeitende Infusionsspritzenpumpe ist daher für verschiedene Arten von Spritzen geeignet, wobei lediglich die entsprechenden Parameter der Spritze in das Rechenwerk eingegeben werden müssen.

Nach dem Merkmal des Patentanspruchs 2 erfolgt eine zeitliche Differenzierung des Signals des Flüssigkeitsdrucks bzw. es wird die Ableitung des Signals des Flüssigkeitsdrucks gebildet. Auf diese Weise kann ein plötzlicher Anstieg des Flüssigkeitsdrucks oder auch das Anstoßen des Kolbens an die Zylinderbrust ermittelt werden. Es wird also der Gradient des Flüssigkeitsdrucks gebildet und ein plötzliches Ansteigen dieses Drucks bewirkt das Abschalten, selbst wenn die absolute Größe des Drucks noch unter dem oberen Druckwert liegt. Der Leerzustand der Spritze wird dadurch früher erkannt als im Falle einer nur druckabhängigen Überwachung. Dies ist insbesondere bei sehr kleinen Infusionsraten und niedriger Vorschubgeschwindigkeit des angetriebenen Halters wichtig, weil bei einer kraftabhängigen oder druckabhängigen Signalerzeugung der Aufbau des Anschlagdrucks des Kolbens an der Zylinderbrust eine erhebliche Zeit erfordert, in der keine Flüssigkeit mehr aus dem Zylinder herausgepreßt wird. Auch ein durch Verschluß der Infusionsleitung hervorgerufener Druckanstieg wird frühzeitig erkannt.

Gemäß Anspruch 3 wird bei Infusionsbeginn der Antrieb bis zum Aufbau des Arbeitsdrucks vorgefahren, wodurch das mechanische Spiel der Antriebseinrichtung des angetriebenen Halters überwunden wird. Die Infusion mit der vorgesehenen Infusionsrate erfolgt erst, wenn der Flüssigkeitsdruck den unteren Druckwert überschritten hat.

Mit den Merkmalen des Anspruchs 4 wird das Bolusvolumen minimiert. Das Bolusvolumen ist dasjenige Volumen, um das sich das Infusionssystem unter Einwirkung des Flüssigkeitsdruckes, bei Vorliegen eines Verschlusses der Infusionsleitung, aufweitet. Wird dieser Verschluß beseitigt, so entspannt sich das System und es kann eine gefährliche Überdosierung eintreten. Nach den Merkmalen des Anspruchs 4 wird der Verschlußdruck durch Zurückfahren des Antriebs abgebaut und eine Bolusgabe somit ausgeschlossen.

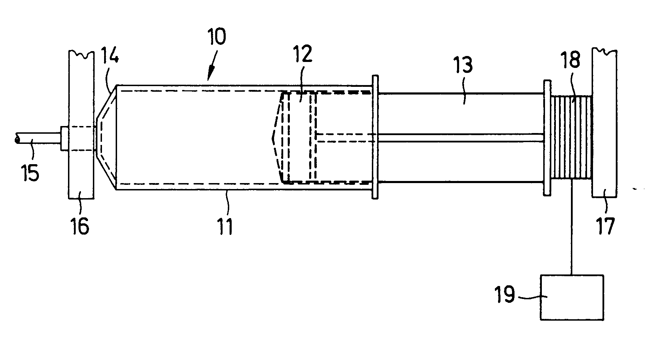

Im folgenden wird unter Bezugnahme auf die einzige Figur der Zeichnung ein Ausführungsbeispiel der Erfindung näher erläutert.

In der Zeichnung ist eine in eine Infusionsspritzenpumpe eingesetzte Spritze schematisch dargestellt.

Die Spritze 10 ist eine Einmalspritze aus Kunststoff mit einem Spritzenzylinder 11, in dem der Spritzenkolben 12 verschiebbar ist. Die Kolbenstange 13 ragt aus dem rückwärtigen Ende des Spritzenzylinders heraus. Am vorderen Ende des Spritzenzylinders 11 befindet sich die Spritzenbrust 14 und ein Hals, an den die zum Patienten führende Infusionsleitung 15 angeschlossen ist.

Die Spritze 10 ist zwischen den beiden Haltern 16 und 17 der Infusionsspritzenpumpe auswechselbar angeordnet. Der Halter 16, der die Spritzenbrust 14 abstützt, ist fest an dem Gerät angebracht, während der Halter 17, der die Kolbenstange abstützt in Richtung auf den Halter 16 bewegbar ist, um den Inhalt der Spritze 10 auszudrücken. Am Halter 17 ist ein Kraftsensor 18 befestigt, gegen den die Kolbenstange 13 drückt und der die Kraft mißt, welche dem Halter 17 vom Kolben 12 entgegengesetzt wird. Der Kraftsensor 18 ist an das Rechenwerk 19 angeschlossen.

Dem Rechenwerk 19 werden die Konstanten: Kolbenfläche A und Reibkraft Fr der Spritze 10 eingegeben. Vom Kraftsensor 18 empfängt das Rechenwerk die Größe Fk der Kolbenkraft. Aus diesen Größen berechnet das Rechenwerk den Flüssigkeitsdruck P nach der Formel

$$P = (Fk - Fr) / A.$$

Der Flüssigkeitsdruck P wird mit einem oberen Druckwert $P_o$ verglichen und der Antrieb der Infusionsspritzenpumpe wird abgeschaltet, wenn P den oberen Druckwert $P_o$ übersteigt. Dies ist der Fall, wenn die Infusionsleitung 15 blockiert oder abgeknickt ist.

Das Rechenwerk 19 führt ferner eine Differenzierung des Wertes P durch und es erzeugt ein Signal, wenn der Wert der Ableitung $P'$ des Flüssigkeitsdrucks einen oberen Grenzwert überschreitet. Auf diese Weise wird ein Anschlag des Kolbens 12 gegen die Spritzenbrust 14 und ein vollständiges Blockieren der Infusionsleitung 15 frühzeitig erkannt, noch bevor der obere Druckwert $P_o$ erreicht ist.

Bei Infusionsbeginn wird der Halter 17 in Richtung auf den Halter 16 schnell vorwärtsbewegt, bis der Flüssigkeitsdruck P einen unteren Grenzwert $P_u$ überschritten hat. Danach beginnt die Infusion mit einer Vorschubgeschwindigkeit des Halters 17, die der vorgesehen Infusionsrate entspricht. Auf diese Weise wird mechanisches Spiel der Antriebseinrichtung des Halters 17 überwunden.

Bei Infusionsunterbrechung wird der Halter 17 vom Halter 16 fort so weit zurückgefahren, bis der Flüssigkeitsdruck einen unteren Druckwert unterschreitet. Auf diese Weise wird das gespeicherte Bolusvolumen, das auf die Elastizität von Spritze und Infusionsleitung zurückzuführen ist, abgebaut, ohne daß die Spritze jedoch Flüssigkeit von außen ansaugt.

## Patentansprüche

1. Verfahren zur Überwachung des Betriebs einer Infusionsspritzenpumpe, die einen festen Halter (16) und einen angetriebenen zweiten Halter (17) zum Ausdrücken einer Spritze (10) aufweist, bei welchem bei Überschreiten einer vorgegebenen Belastung der Spritze Alarm augelöst wird,
**dadurch gekennzeichnet,** daß die von der Spritze (10) auf einen der Halter (17) ausgeübte Kraft (Fk) gemessen und der Meßwert einem Rechenwerk (19) zugeführt wird und daß das Rechenwerk (19) unter Berücksichtigung der ihm eingegebenen Größen von Reibkraft (Fr) und Querschnittsfläche (A) der Spritze (10) den Flüssigkeitsdruck (P) ermittelt und ein Signal erzeugt, wenn dieser Flüssigkeitsdruck einen oberen Druckwert ($P_o$) überschreitet.

2. Verfahren nach Anspruch 1, dadurch gekennzeichnet, daß das Rechenwerk eine zeitliche Differenzierung des Flüssigkeitsdrucks ausführt und ein Signal erzeugt, wenn der Wert des Gradienten des Flüssigkeitsdrucks einen oberen Grenzwert überschreitet.

3. Verfahren nach Anspruch 1 oder 2, dadurch gekennzeichnet, daß der angetriebene Halter (17) nach dem Einschalten schnell vorgefahren wird, bis der Flüssigkeitsdruck (P) einen unteren Druckwert ($P_u$) überschreitet, und anschließend mit einer der vorgesehenen Infusionsrate entsprechenden Geschwindigkeit weiterbewegt wird.

4. Verfahren anch einem der Ansprüche 1 bis 3, dadurch gekennzeichnet, daß bei Infusionsunterbrechung oder -abschaltung der angetriebene Halter (17) zurückbewegt wird, bis der Flüssigkeitsdruck (P) einen unteren Druckwert unterschreitet.

## Claims

1. Method for monitoring the operation of an infusion syringe pump having a fixed holder (16) and a driven second holder (17) for squeezing out a syringe (10), wherein an alarm is triggered when a predetermined load on the syringe is exceeded,

characterised in

that the force (Fk) exerted by said syringe (10) on one of said holders (17) is measured and that said measured value is supplied to a calculator (19) and that said calculator (19), dependent on the values of the frictional force (Fr) and the cross-sectional surface (A) of said syringe (10) inputted therein, determines the liquid pressure (P) and generates a signal when said liquid pressure exceeds an upper pressure value ($P_o$).

2. Method as defined in claim 1, characterised in that said calculator performs a time-differentiation of said liquid pressure and generates a signal when the value of the gradient of said liquid pressure exceeds an upper limit value.

3. Method as defined in claim 1 or 2, characterised in that, after switching on, said driven holder (17) is advanced fast until said liquid pressure (P) exceeds a lower pressure value ($P_u$) and that, thereafter, it is moved on at a speed corresponding to a desired infusion rate.

4. Method as defined in one of claims 1 to 3, characterised in that, upon an interruption or a switch-off of the infusion, said driven holder (17) is moved back until said liquid pressure (P) decreases below a lower pressure value.

## Revendications

1. Procédé pour la surveillance du fonctionnement d'une pompe à seringue d'injection, qui présente un support fixe (16) et un second support entraîné (17) servant à éjecter le contenu d'une Seringue (10), et selon lequel une alarme est déclenchée lorsque la charge appliquée à la seringue dépasse une valeur prédéterminée,
caractérisé en ce que la force (Fk) exercée par la seringue (10) sur un des supports (17) est mesurée et la valeur de mesure est envoyée à une unité de calcul (19), et, sur la base des valeurs, qui sont envoyées à l'unité de calcul (19), de la force de frottement (Fr) et de la surface en coupe transversale (A) de la seringue (10), l'unité de calcul détermine la pression (P) du liquide et produit un signal lorsque cette pression du liquide dépasse une valeur de pression supérieure ($P_o$).

2. Procédé selon la revendication 1, caractérisé en ce que l'unité de calcul exécute une différenciation dans le temps de la pression du liquide et produit un signal lorsque la valeur du gradient de la pression du liquide dépasse une valeur limite supérieure.

3. Procédé selon la revendication 1 ou 2, caractérisé en ce que le support entraîné (17) avance rapidement après la mise en service jusqu'à ce que la pression (P) du liquide dépasse une valeur de pression inférieure ($P_u$), puis consécutivement est entraîné avec une vitesse qui correspond au débit d'injection prévu.

4. Procédé selon l'une des revendications 1 à 3, caractérisé en ce que dans le cas d'une interruption ou d'une mise hors service de l'injection, le support entraîné (17) est ramené en arrière jusqu'à ce que la pression (P) du liquide tombe au-dessous d'une pression inférieure.